# FASCICULE DE BREVET EUROPEEN

(11) **EP 2 240 080 B1**
(45) Date de publication et mention de la délivrance du brevet: **08.02.2012**
(21) Numéro de dépôt: 08872415.8
(22) Date de dépôt: 03.12.2008
(51) Int. Cl.: A61B 6/14, A61B 6/03

(54) **APPAREIL DE RADIOLOGIE DENTAIRE ET PROCEDE ASSOCIE**
ZAHNRÖNTGENGERÄT UND ENTSPRECHENDES VERFAHREN
DENTAL X-RAY APPARATUS AND ASSOCIATED METHOD

(30) Priorité: 03.12.2007 FR 0759516
(43) Date de publication de la demande: 20.10.2010
(73) Titulaire: Trophy, 77437 Croissy Beaubourg (FR)
(72) Inventeur: LOUSTAUNEAU Vincent, F-94120 Fontenay-Sous-Bois (FR); BOTHOREL Sylvie, F-75013 Paris (FR); MAURY Colombe Isabelle, F-77330 Ozoir-la-Ferriere (FR)
(74) Mandataire: Petit, Maxime
(86) Numéro de dépôt international: PCT/FR2008/001680
(87) Numéro de publication internationale: WO 2009/101283

(56) Documents cités:
- EP-A- 1 609 419
- WO-A-2004/075118
- WO-A-2006/013325
- US-A1- 2004 258 195
- US-A1- 2005 063 507
- US-A1- 2007 081 624

## Description

L'invention concerne un appareil de radiologie dentaire et un procédé associé.

Dans le domaine de la radiologie dentaire on connaît des appareils de radiologie qui comprennent un générateur de rayons X et un capteur de rayons X montés chacun sur un bras d'une structure en forme d'arceau.

Lorsque l'on souhaite effectuer une radiographie de la mâchoire d'un patient, celui-ci est installé en position assise sous l'arceau et sa tête est placée entre le générateur de rayons X et le capteur proche de la tête. Les rayons X sont émis par la source sous la forme d'un cône dirigé vers la tête. Le capteur reçoit les rayons ayant irradié la tête du patinent, les convertit en signaux électriques et fournit en sortie un signal d'image de la tête irradiée.

L'arceau pivote autour d'un axe de rotation vertical sur 360° pour pouvoir obtenir plusieurs signaux d'image de la tête du patient sous différents angles.

Par exemple, on peut obtenir un signal d'image ou cliché pour chaque degré de rotation de l'arceau.

Jusqu'à présent, lorsque l'on souhaite pratiquer un examen en trois dimensions d'un objet, tel qu'une demi-arcade dentaire, une rotation de 360° de l'arceau portant le capteur et le générateur est effectuée autour de l'objet.

Si l'on souhaite augmenter la largeur du volume reconstruit par radiographie, la largeur du capteur doit être augmentée en conséquence.

Or, une augmentation de la taille du capteur se traduit par un surcoût important.

Par exemple, le volume qu'il est possible de reconstituer à partir d'un capteur plan de dimensions 5 cm x 6 cm est d'environ 3,2 cm x 4 cm, ramené dans le plan de l'objet. Les dimensions du volume reconstitué tiennent compte de la géométrie conique du faisceau de rayons X et des distances respectives entre le point d'émission des rayons (foyer de la source ou point de l'anode sur lequel est focalisé le faisceau d'électrons), l'objet et le capteur.

Un tel volume reconstruit est insuffisant pour reconstituer une taille d'image correspondant à une demi-arcade dentaire.

Il serait par conséquent intéressant de pouvoir augmenter la taille de l'image reconstruite à partir d'un capteur de dimensions données.

La présente invention a ainsi pour objet un appareil de radiologie dentaire conforme à la revendication 1.

Le décalage du capteur doit être significatif pour pouvoir augmenter le volume de l'objet reconstruit, sans toutefois être trop élevé afin de ne pas acquérir de portions d'images inutiles (sans rapport avec l'objet d'intérêt) et de ne pas occulter de zones de l'objet d'intérêt.

Ainsi, dans chaque position angulaire du capteur et du générateur, ceux-ci coopèrent pour acquérir une image d'une partie décalée latéralement de l'objet, là où, dans l'art antérieur, l'image capturée était centrée sur l'objet.

En se déplaçant autour de l'objet, grâce à cet agencement, au final une plus grande étendue latérale (considérée perpendiculairement à l'axe longitudinal qui relie le capteur au générateur en passant par l'axe de rotation de ceux-ci) de l'objet est capturée par l'ensemble générateur et capteur.

Ceci permet donc de reconstituer un plus grand volume d'objet qu'auparavant en conservant le même capteur.

On notera par ailleurs que le générateur de rayons X n'illumine que la partie de l'objet située dans malignement du capteur décalé, dans une position angulaire donnée.

Or, comme l'ensemble générateur et capteur tourne, le cône de rayons X émis par le générateur balaye des zones ou parties différentes de l'objet au cours des rotations successives, au lieu de toujours balayer la zone centrale de cet objet.

Ainsi, une même zone de l'objet reçoit une dose de rayons X inférieure à la dose reçue par la zone centrale balayée par le cône de rayons X dans l'art antérieur.

On notera toutefois que le diaphragme ou collimateur du générateur de rayons X est lui aussi décalé de façon correspondante pour que le faisceau de rayons X émis illumine la zone d'intérêt de l'objet sur au moins une partie de la surface du capteur décalé. Le faisceau collimaté est donc lui aussi décalé afin d'être centré par exemple sur le capteur.

L'axe central du faisceau relie ainsi le générateur au centre du capteur et est donc également décalé par rapport à l'axe longitudinal reliant perpendiculairement le générateur au capteur et passant par l'axe de rotation.

En outre, le rayonnement diffusé à l'objet est utilisé plus efficacement que dans fart antérieur.

Par ailleurs, le faisceau est collimaté de telle façon qu'une zone périphérique de la surface active du capteur décalé soit peu illuminée par ce faisceau par rapport à la partie centrale de la surface active et qui constitue la plus grande partie de cette surface.

Cette zone ou frange peu éclairée permet de s'assurer que l'intensité maximale du faisceau de rayons X illumine la plus grande partie de la surface active du capteur et, qu'au-delà, c'est-à-dire sur la zone périphérique, l'intensité du faisceau est considérablement réduite. La zone périphérique non illuminée assure ainsi une fonction de sécurité vis-à-vis du rayonnement X (radioprotection).

En pratique, l'intensité moyenne du rayonnement reçue par la zone périphérique est comprise entre 25 et 35% de l'intensité moyenne du rayonnement reçue dans la partie restante du capteur pour assurer efficacement une fonction de radioprotection.

On notera que le faisceau ainsi collimaté est obtenu par ajustement des moyens de collimation qui, par exemple, peuvent prendre l'apparence d'une ou de plusieurs fentes de collimation.

La largeur minimale de la zone périphérique est celle qui permet d'obtenir au bord du capteur une intensité de rayonnement suffisamment réduite par rapport à l'intensité maximale reçue par la partie centrale de la surface active.

Selon une caractéristique, le capteur et le générateur sont aptes à se déplacer en rotation dans un plan dit de rotation qui est traversé par l'axe de rotation.

Selon une caractéristique, le centre du capteur est positionné transversalement par rapport à la projection de l'axe longitudinal sur la surface active du capteur à une distance qui, mesurée dans le plan de rotation, est au maximum égale à la différence entre la demi-largeur du capteur et une largeur suffisante pour laisser la zone périphérique de la surface active du capteur faiblement illuminée par rapport au reste de la surface active, la largeur du capteur étant la dimension mesurée dans le plan de rotation perpendiculairement à l'axe longitudinal.

Ce décalage maximal procure l'efficacité maximale liée au décalage, notamment en termes de volume de reconstruction et de zone irradiée.

Selon une caractéristique, le centre du capteur est décalé d'une distance comprise entre le quart de la largeur du capteur et la distance maximale précitée qui est légèrement inférieure à la demi-largeur du capteur, compte-tenu de la zone périphérique qui est peu illuminée.

La largeur de la zone périphérique est appropriée pour assurer cette fonction de sécurité en tenant compte de la précision d'installation et de positionnement des différents éléments : capteur, moyens de collimation et générateur, précision du faisceau.

Cette largeur représente ainsi par exemple plusieurs pixels entre la partie de la surface active illuminée du capteur et le bord de celui-ci.

Selon une caractéristique, l'agencement des moyens de collimation et du capteur est tel que le faisceau collimaté illuminant la surface active du capteur est délimité par un bord qui est placé au plus près de la projection de l'axe sur la surface active, à une distance minimale qui permet d'obtenir une zone de recouvrement minimale lors de la rotation du générateur et du capteur.

On s'assure ainsi d'un volume de recouvrement minimal avec l'objet à illuminer au cours de la rotation du générateur et du capteur pour pouvoir reconstruire une représentation volumique de cet objet qui soit exempte d'artéfacts.

En pratique, la distance minimale considérée est de l'ordre de deux pixels, les pixels étant ceux de la matrice de pixels formant la surface active du capteur.

Cette distance est généralement inférieure à la largeur de la zone périphérique non illuminée du capteur.

Selon une caractéristique, la pente d'anode du générateur est modifiée en fonction de la position décalée du capteur afin de rendre plus uniforme le profil du rayonnement illuminant la surface active du capteur ainsi décalé. Cette nouvelle configuration de la pente d'anode complète la nouvelle géométrie obtenue avec le capteur décalé et les moyens de collimation ajustés comme mentionné plus haut.

Selon une caractéristique particulière, l'angle formé par la pente d'anode du générateur et l'axe longitudinal est ouvert dans la direction vers laquelle le capteur est décalé.

L'intensité du rayonnement produit par le générateur et éclairant le capteur décalé est ainsi augmentée et le profil de ce rayonnement qui atteint le capteur est rendu plus homogène.

En pratique, la valeur de l'angle précité augmente.

Selon une caractéristique, le plan de rotation est horizontal.

Selon une caractéristique, l'axe de rotation est vertical.

L'invention a également pour objet un procédé de reconstitution d'une représentation volumique d'un objet irradié par des rayons X à partir d'images radiographiques dentaires planes conforme à la revendication 8.

On entend par image radiographique plane une image radiographique de l'objet en 3D projetée dans un plan.

Ce procédé présente les mêmes avantages que ceux de l'appareil brièvement exposé ci-dessus et ils ne seront donc pas rappelés ici.

Selon une caractéristique, le capteur et le générateur sont aptes à se déplacer en rotation dans un plan dit de rotation qui est traversé par l'axe de rotation.

Selon une caractéristique, le centre du capteur est positionné transversalement par rapport à la projection de l'axe longitudinal sur la surface active du capteur à une distance qui, mesurée dans le plan de rotation, est au maximum égale à la différence entre la demi-largeur du capteur et une largeur suffisante pour laisser la zone périphérique de la surface active du capteur faiblement illuminée par rapport au reste de la surface active, la largeur du capteur étant la dimension mesurée dans le plan de rotation perpendiculairement à l'axe longitudinal.

Selon une caractéristique, le centre du capteur est décalé d'une distance comprise entre le quart de la largeur du capteur et la distance maximale.

Selon une caractéristique, l'agencement des moyens de collimation et du capteur est tel que le faisceau collimaté illuminant la surface active du capteur est délimité par un bord qui est placé au plus près de la projection de l'axe sur la surface active, à une distance minimale qui permet d'obtenir une zone de recouvrement minimale lors de la rotation du générateur et du capteur.

Selon une caractéristique, la pente d'anode du générateur est modifiée en fonction de la position décalée du capteur afin de rendre plus uniforme le profil du rayonnement illuminant la surface active du capteur ainsi décalé.

Selon une caractéristique, l'angle formé par la pente d'anode du générateur et l'axe longitudinal est ouvert dans la direction vers laquelle le capteur est décalé.

Selon une autre caractéristique, le procédé comporte une étape de déplacement simultané du capteur et du générateur en rotation autour de l'axe de rotation, dans plusieurs positions angulaires successives, et pour chacune de ces positions angulaires successives, le procédé comporte une étape de fourniture, par le capteur, d'un signal représentatif d'une image radiographique plane de l'objet irradié suivant cette position angulaire, l'ensemble des signaux fournis par le capteur pour l'ensemble des positions angulaires successives contenant la totalité des données nécessaires à la reconstruction d'une représentation volumique de l'objet.

Selon une caractéristique, le déplacement du capteur et du générateur est effectué sur un tour de rotation, ce qui permet d'obtenir suffisamment de données pour reconstituer la totalité de l'objet ou d'une zone d'intérêt de celui-ci en trois dimensions.

Selon une caractéristique, le procédé comporte une étape de traitement des signaux fournis par le capteur pour l'ensemble des positions angulaires successives afin de reconstruire la représentation volumique de l'objet.

Selon une caractéristique, le traitement comprend une étape de filtrage permettant de différencier le bruit associé aux signaux de l'information utile présente dans ces signaux.

Selon une caractéristique, le filtrage comprend une étape de décomposition de façon indépendante des différentes bandes de fréquence présentes dans les signaux.

Selon une caractéristique, le traitement comprend une étape de filtrage multiéchelle à décomposition de type pyramidal.

Selon une caractéristique, le traitement comprend une étape de pondération des données qui sont issues des différents signaux et proviennent des parties de l'objet successivement illuminées par le faisceau de rayons X au cours des rotations successives, la pondération étant ajustée en fonction de la présence ou non des parties illuminées de l'objet dans une zone de ce dernier appelée zone de recouvrement et qui est toujours illuminée par le faisceau au cours des rotations successives.

Ainsi, on affecte des coefficients de pondération différents aux données selon leur position par rapport aux zones successivement balayées par le cône de rayons X.

Cette pondération prend en compte le décalage latéral du capteur tel que décrit plus haut.

D'autres caractéristiques et avantages apparaîtront au cours de la description qui va suivre, donnée uniquement à titre d'exemple non limitatif, et faite en référence aux dessins annexés, sur lesquels :
- la figure 1 est une vue schématique générale en perspective d'un appareil de radiologie dentaire selon l'invention ;
- la figure 2 est une vue schématique de dessus montrant l'agencement du capteur et du générateur selon fart antérieur ;
- les figures 3a et 3b sont des vues schématiques de dessus montrant l'agencement du capteur et du générateur selon l'invention dans deux positions angulaires ;
- la figure 3c illustre la modification de la pente d'anode pour une configuration du capteur excentrée ;
- la figure 3d est une vue schématique montrant l'intensité du faisceau de rayons X en fonction de son inclinaison par rapport à l'axe longitudinal 34 ;
- la figure 3e est une vue schématique partielle agrandie de la figure 3a et montrant la zone de recouvrement ;
- la figure 3f représente de façon schématique la position décalée du capteur pour obtenir une zone de recouvrement minimale ;
- la figure 4 est une vue schématique des opérations de traitement des données fournies par le capteur.

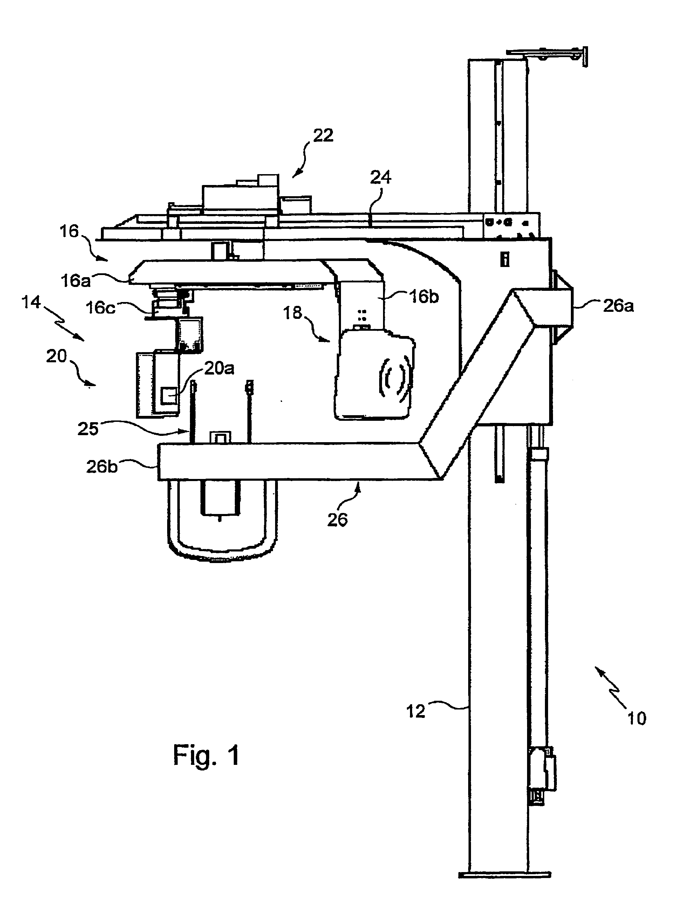

Comme représenté à la figure 1 et désigné par la référence générale notée 10, un appareil de radiologie dentaire selon l'invention est un appareil du type à tomographie numérisée à faisceau conique (connu en terminologie anglo-saxonne sous le terme CBCT, acronyme signifiant « Cone Beam Computed Tomography »). Cet appareil permet d'acquérir des images volumiques d'un objet. L'appareil comprend un bâti 12 fixe, par exemple une potence verticale, sur laquelle est montée une unité radiographique tournante 14 qui va maintenant être décrite.

Cette unité comprend une structure mobile 16 en forme de C couché (arceau) comportant une poutre centrale horizontale 16a qui constitue le corps du C et deux bras verticaux 16b et 16c descendant à partir de la poutre horizontale et qui constituent chacun les deux branches du C.

Une source ou générateur de rayons X 18 est monté(e) de manière fixe sur le bras 16b, tandis qu'un capteur de rayons X 20 est monté sur le bras 16c.

Le générateur 18 et le capteur 20 sont ainsi disposés en vis-à-vis l'un de l'autre et sont dans une relation géométrique fixe l'un par rapport à l'autre.

La structure 16 qui sert de support au générateur 18 et au capteur 20 constituant le coeur de l'unité radiographique tournante 14 est reliée à une table 22 disposée au-dessus de la structure 16 et qui est mobile en X et Y.

Plus particulièrement, cette table est montée sur une poutre horizontale 24 fixée au bâti vertical 12.

Cette table est susceptible de se déplacer dans les directions X et Y dans un plan horizontal, permettant ainsi d'effectuer une rotation complète (360°) autour d'un axe de rotation vertical non représenté sur cette figure.

Cette table permet de positionner le centre de rotation (et l'axe de rotation) de la structure sur un objet à radiographier, notamment une zone d'intérêt du patient, sans que ce dernier n'ait besoin de se déplacer.

On notera que la structure 16 reliée à la table 22 est donc susceptible d'effectuer un mouvement de rotation autour d'un axe de rotation vertical positionné par rapport au patient.

Au cours de ce mouvement de rotation, le générateur 18 et le capteur 20 ne bougent pas l'un par rapport à l'autre.

On notera que la table 22 permet également d'effectuer des trajectoires panoramiques programmables lorsque l'appareil est utilisé dans cette application.

L'appareil de radiologie 10 comporte également un bras inférieur 26 fixé par une extrémité 26a au bâti 12. L'extrémité libre 26b du bras est équipée d'un dispositif de positionnement 25 permettant d'immobiliser la tête du patient pendant la prise de clichés radiographiques, lors du fonctionnement de l'appareil. La tête est ainsi intercalée entre le générateur 18 et le capteur 20.

Le générateur de rayons X 18 comprend plus particulièrement un tube de rayons X, par exemple à anode fixe, et dont la taille du foyer est, par exemple, égale à 0,5 mm.

En outre, ce générateur comprend des moyens de collimation pour collimater un faisceau de rayons X généré par le générateur 18. Ces moyens comprennent par exemple une fenêtre ou fente de collimation (diaphragme) qui est plombée et dimensionnée pour produire un faisceau conique de rayons X destiné à illuminer une partie de la tête du patient (par exemple la mâchoire) et le capteur disposé derrière.

On notera que la largeur de la fente est réglable pour ajuster la largeur du faisceau et peut également être orientée différemment afin d'orienter le faisceau dans une direction donnée. La fente peut par exemple être décalée latéralement.

Selon une variante non représentée, les moyens de collimation peuvent comprendre plusieurs fentes de forme et/ou de taille diverses qui sont commutables devant le générateur et permettent d'ajuster la largeur du faisceau et/ou sa forme et/ou son orientation géométrique à la position décalée du capteur.

Le capteur 20 est assujetti à un bras 16c motorisé qui permet de faire pivoter autour d'un axe vertical l'équipement porté par ce bras et, selon l'application choisie, de positionner en face du générateur soit le capteur 20 destiné à un examen en trois dimensions de l'objet à irradier, soit une cassette en barrette (non représentée) destinée à un examen panoramique.

On notera que le capteur 20 utilisé pour la reconstitution d'un objet en trois dimensions (ex. : tête du patient) est un capteur plan.

Ce capteur est apte, d'une part, à recevoir un rayonnement X provenant du générateur 18 et ayant illuminé l'objet placé entre le capteur et le générateur et, d'autre part, à transformer ce rayonnement en un signal électrique représentatif d'une image radiographique de cet objet.

Plus particulièrement, le capteur comprend par exemple :
- un convertisseur qui est apte à convertir en rayonnement visible, le rayonnement X reçu par le capteur ; ce convertisseur est par exemple un scintillateur réalisé en lodure de Césium, et
- un détecteur du rayonnement visible transformé en provenance du convertisseur et qui fournit, en sortie du capteur, le signal électrique représentatif d'une image radiographique de l'objet.

Une plaque de fibres optiques dopée en particules métalliques afin d'absorber le rayonnement X non converti est par exemple placée entre le scintillateur et le détecteur.

Cette plaque est par exemple celle commercialisée par la société Hammamatsu sous la référence commerciale XR5, ou celle de la société Schott référencée 47A.

On notera que le détecteur est par exemple un détecteur CMOS qui est préférable à un détecteur de type CCD pour effectuer de la tomographie numérisée à faisceau conique. En effet, la Demanderesse s'est aperçue qu'un tel composant est plus approprié lorsque l'on souhaite réduire la dose de rayonnement compte tenu du grand nombre de projections obtenues lors d'une rotation de 360° de l'ensemble capteur et générateur. L'utilisation d'un détecteur CMOS à matrice active est avantageuse.

Plus particulièrement, une matrice de pixels active réalisée en technologie biCMOS et dotée d'un taux élevé de remplissage des pixels (connu en terminologie anglo-saxonne sous le terme « fill factory ») peut être avantageusement utilisée. La matrice de pixels possède par exemple une taille de pixels de l'ordre de 120 microns et permet de lire rapidement des images capturées, par exemple en 15 millisecondes.

En diminuant la taille du détecteur par rapport à la taille de l'objet à reconstruire on s'assure de pouvoir fabriquer le détecteur en technologie CMOS compte tenu de la taille des "*wafers*" disponibles. De tels détecteurs CMOS disposent d'un rapport signal sur bruit élevé.

La taille du capteur plan est par exemple de 5 cm x 6 cm.

A titre de variante, lorsque l'on souhaite reconstruire un objet de grand volume, un capteur de type TFT peut par exemple être avantageusement utilisé.

Une couche scintillatrice, par exemple en oxysulfure de Gadolinium ou en lodure de Césium, est déposée sur le détecteur pour assurer la conversion du rayonnement X en rayonnement visible.

On a représenté sur la figure 1 la surface optiquement active 20a du capteur 20.

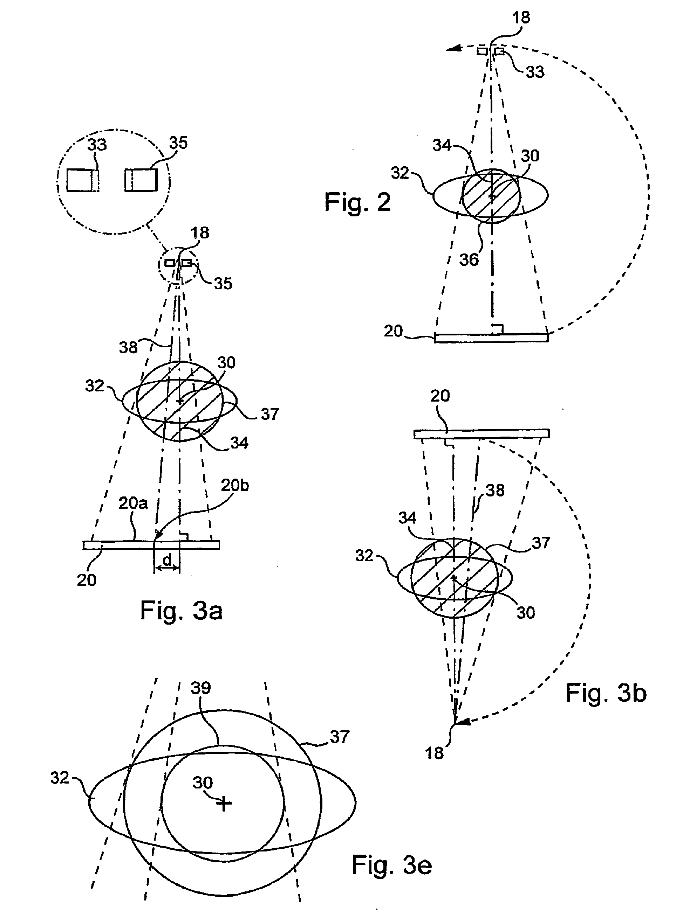

La figure 2 illustre l'agencement classique d'une source de rayons X et du capteur de rayons X associé par rapport à la position de l'objet à irradier placé entre eux.

On voit ainsi que l'axe de rotation du capteur 20 et du générateur 18, matérialisé sur la figure 2 par le point référencé 30 et qui est positionné par rapport à l'objet 32 à illuminer, est placé sur l'axe d'alignement 34 (axe longitudinal) qui relie perpendiculairement le générateur 18 au capteur 20.

Cet axe 34 constitue l'axe du faisceau collimaté de rayons X schématisé sur cette figure, émis par le générateur 18 et qui vient frapper le centre du capteur 20 après avoir rencontré l'axe vertical 30.

Le faisceau de rayons X est collimaté par une fente 33 centrée sur l'axe central 34 du faisceau.

Dans cette configuration illustrant l'art antérieur, on constate qu'au cours du mouvement de rotation du générateur et du capteur autour de l'objet à illuminer 32, le faisceau de rayons X émis par le générateur balaye toujours la même zone centrale 36 de l'objet 32.

Les zones latérales de l'objet 32 qui sont disposées de part et d'autre de la zone centrale 36 ne sont pas illuminées et donc l'information qu'elles contiennent n'est pas capturée par le faisceau de rayons X.

On notera que le générateur 18 et le capteur 20 se déplacent tous deux dans un plan de rotation perpendiculaire à l'axe de rotation vertical 30 et qui correspond au plan de la figure 2.

Les figures 3a et 3b illustrent le positionnement de l'ensemble capteur et générateur d'un appareil de radiologie selon l'invention dans deux positions angulaires différentes séparées de 180° l'une de l'autre.

Sur la figure 3a qui est une vue dans un plan horizontal analogue à celui de la figure 2, le capteur 20 présente un décalage latéral dans ce plan par rapport à la position qu'il occupe sur la figure 2 (décalage obtenu par une translation du capteur). La fente de collimation 35 du générateur de rayons X 18 est également décalée latéralement de façon correspondante pour que le faisceau collimaté illumine la majorité de la surface active du capteur décalé et soit centré sur celle-ci. Le décalage de la fente 35 est illustré sur la partie agrandie de la figure 3a où l'on voit également, en pointillés, la position de la fente 33 de la figure 2.

Avec un tel décalage latéral du capteur et du générateur, l'axe central 38 du cône de rayons X collimaté par la fente du générateur 18 et qui vient frapper le capteur 20 en son centre (cet axe peut être considéré comme l'axe d'alignement du capteur et du générateur), n'est plus concourant avec l'axe de rotation vertical 30 comme sur la figure 2 mais passe à côté de celui-ci (figure 3a).

On notera que le capteur 20 est orienté de telle façon que l'axe longitudinal 34 qui relie perpendiculairement le générateur 18 à la surface active 20a du capteur 20 passe par l'axe de rotation 30. La largeur du capteur est la dimension du capteur mesurée dans le plan de rotation de la figure 3a, perpendiculairement à l'axe 34.

Le décalage du capteur peut ainsi être défini comme étant le décalage transversal d du centre du capteur 20b par rapport à la projection de l'axe longitudinal 34 sur la surface active 20a.

Comme représenté sur les figures 3a et 3b, une petite partie 20c de la surface active du capteur est séparée, par le faisceau dont les bords sont illustrés en pointillés, de la partie centrale où est placé le centre 20b du capteur et qui reçoit l'intensité maximale du rayonnement. La partie 20c qui forme une zone périphérique (de dimensions réduites par rapport à celles de la partie restante du capteur) entourant la partie centrale reçoit un rayonnement de faible intensité moyenne par rapport au rayonnement d'intensité moyenne reçu par la partie centrale et qui est par exemple compris entre 25 et 35% de cette intensité moyenne.

A titre d'exemple, la largeur est égale à 10 pixels.

La géométrie de cette zone est obtenue en réglant la largeur entre les bords espacés de la fente 35 ou, selon une variante, en sélectionnant une fente de collimation de largeur appropriée parmi plusieurs fentes possibles.

L'appareil de radiologie dentaire ainsi doté d'un capteur décalé et de moyens de collimation ajustés de façon à produire un faisceau collimaté projetant sur la surface active du capteur l'image des moyens de collimation (ex : image des bords de la fente 35) permet d'optimiser la reconstruction volumique d'objet (mâchoire ou partie de mâchoire), tout en assurant une fonction de protection vis-à-vis du rayonnement X.

Dans le cas où l'on procède à un examen en 3D, on peut orienter avantageusement le générateur afin d'accroitre l'intensité du faisceau émis et d'améliorer l'homogénéité de l'éclairement produit par le faisceau.

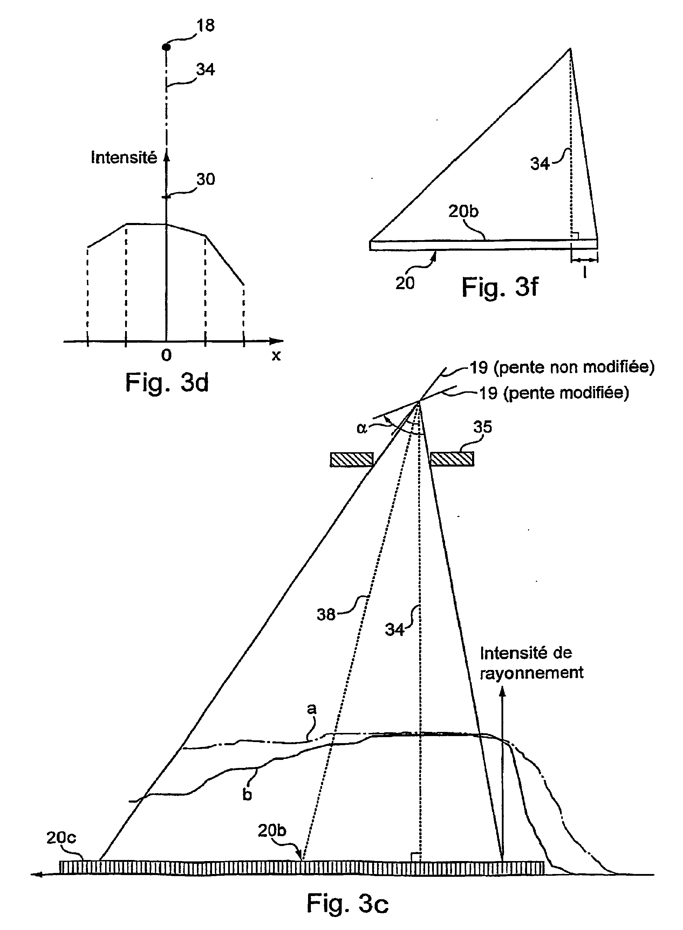

Cette orientation s'effectue en augmentant l'angle α formé par la pente d'anode 19 et l'axe 34 (fig. 3c).

Dans la configuration du capteur décalé de la distance maximale l'angle α passe de 5° (pente d'anode non modifiée) à 7°.

On notera que, de façon générale, la nouvelle valeur de l'angle α dépend du décalage du capteur. Plus particulièrement, cette nouvelle valeur correspond à l'Arctangente du rapport de la distance de décalage sur la distance entre le foyer du générateur et la surface du capteur.

Comme représenté sur la figure 3c, l'augmentation de l'angle α permet d'étendre la surface irradiée et d'uniformiser le profil du rayonnement obtenu sur la surface active du capteur décalé. Le profil optimisé est représenté par la courbe a, tandis que la courbe b illustre le profil non uniforme du rayonnement obtenu avec une pente d'anode non modifiée. On notera que l'intensité du rayonnement éclairant la zone périphérique bordant le capteur est fortement réduite par rapport à l'intensité dans la partie complémentaire du capteur.

La figure 3d illustre l'intensité du faisceau de rayons X généré par la source 18 dans le plan de la surface active du capteur 20, en fonction de l'inclinaison (axe X) de ce faisceau par rapport à l'axe longitudinal 34. Cette intensité est mesurée dans le plan du capteur (non représenté sur cette figure) et est maximale à gauche de l'axe 34, c'est-à-dire pour une orientation angulaire du faisceau formant un angle non nul avec l'axe 34. On constate ainsi qu'en orientant la pente d'anode du générateur comme décrit ci-dessus et illustré sur la figure 3c, l'intensité maximale du faisceau suit le déplacement du capteur.

La valeur du décalage d du capteur est limitée par la nécessité d'une zone de recouvrement entre toutes les zones ou parties de l'objet 32 balayées par le faisceau de rayons X au cours des rotations successives du capteur et du générateur. Cette zone de recouvrement est représentée par la référence 39 sur la figure 3e (vue partielle agrandie de la figure 3a) qui correspond, dans le plan de rotation de la figure, à un cercle centré sur la référence 30. Toutefois, il convient de noter que la zone de recouvrement est tridimensionnelle et qu'il s'agit d'un cylindre de révolution d'axe de révolution 30.

Afin d'illustrer ce qui précède, on a représenté sur la figure 3f un faisceau de rayons X illuminant le capteur dans une position décalée.

Pour des raisons de simplification, l'objet à radiographier n'a pas été représenté, ni la zone périphérique de sécurité vis-à-vis du rayonnement.

Dans cette configuration décalée, le décalage maximal qu'il est possible d'obtenir correspond à la demi-largeur du capteur diminuée de la largeur l qui représente la distance minimale pour pouvoir obtenir une zone de recouvrement minimale mais suffisante pour être dénuée d'artéfacts lors de la reconstruction volumique de l'objet.

Cette largeur est la distance minimale entre la projection de l'axe 34 sur la surface du capteur et le bord le plus proche du faisceau, bord qui est du côté opposé à celui en direction duquel le capteur a été décalé.

En pratique, cette largeur est d'au moins deux pixels.

Ainsi, le centre 20b du capteur est décalé transversalement de la projection de l'axe longitudinal 34 sur la surface active 20a du capteur d'une distance qui est inférieure à la demi-largeur du capteur (U2) et qui est au plus égale à L/2-l pour les raisons exposées ci-dessus liées au recouvrement minimal.

Compte-tenu de la présence de la zone périphérique 20c de la surface active du capteur, le capteur peut être décalé au maximum de la distance L/2-l diminuée de la largeur de cette zone 20c lorsque l'on souhaite augmenter le plus possible la surface d'irradiation pour une taille de capteur donnée. On s'assure ainsi d'une zone de recouvrement minimale.

D'un point de vue pratique, le capteur est par exemple décalé d'une distance comprise entre le quart de largeur du capteur et la distance maximale précitée inférieure à la moitié de cette largeur.

Par exemple, pour le capteur précité dont la largeur est de 5 cm, le décalage latéral d est par exemple de 2 cm.

Toutefois, un décalage plus petit que le quart de la largeur du capteur (exemple :1/8) peut également être envisagé.

La figure 3b représente l'agencement du capteur et du générateur après avoir effectué un demi-tour autour de l'objet 32 à irradier et l'on constate ainsi que la zone balayée par le cône de rayons X émis par le générateur n'est pas identique à la zone balayée sur la figure 3a.

On notera que ces deux zones se recouvrent autour de l'axe 30 et qu'elles se combinent en un volume balayé de plus grandes dimensions que celles de la zone 36 de l'art antérieur illustré sur la figure 2.

Le volume qu'il est possible de reconstruire avec l'agencement des figures 3a-e est représenté par la zone référencée 37 dans le plan de ces figures. Cette zone 37 est bien entendu tridimensionnelle et a la forme d'un cylindre de révolution d'axe de révolution 30.

L'invention permet donc de couvrir une plus grande étendue de l'objet à irradier pour une même taille de capteur et donc d'augmenter le volume des données qu'il est possible de reconstruire par radiographie en trois dimensions.

L'invention permet ainsi, lorsqu'elle est appliquée au domaine du dentaire, de reconstituer en trois dimensions une zone de la mâchoire d'un patient telle qu'une demi-arcade dentaire avec un capteur de taille réduite par rapport à l'art antérieur.

A titre d'exemple, un décalage latéral de 2 cm du capteur de dimensions 5 cm x 6 cm permet de reconstruire un volume d'objet ayant, en projection dans un plan, des dimensions égales à 5,8 cm x 4 cm. En l'absence de décalage, les dimensions (projetées dans un plan) du volume qu'il est possible de reconstruire ne seraient que 3,2 cm x 4 cm.

On notera que les dimensions du volume reconstitué tiennent compte de la géométrie du faisceau de rayons X et des distances respectives entre le point d'émission des rayons, l'objet et le capteur.

On va maintenant décrire l'acquisition et le traitement des données par l'unité de radiographie tournante de la figure 1 et des figures 3a et 3b lors d'une rotation d'un tour de l'ensemble formé du générateur 18 et du capteur 20 dans la nouvelle configuration décrite ci-dessus et illustrée sur les figures 3a à 3f et notamment sur la figure 3c.

Ainsi, un objet à radiographier tel que la tête d'un patient est interposé entre le générateur 18 et le capteur 20 de la figure 1 et l'ensemble formé du générateur et du capteur adopte une pluralité de positions angulaires successives obtenues par rotations successives de cet ensemble autour de l'axe de rotation vertical 30. Le faisceau de rayons X collimaté émis par le générateur illumine à chaque position une zone différente de l'objet à illuminer. Ce rayonnement est modifié par sa rencontre avec l'objet et le capteur placé dans l'alignement du générateur reçoit ce rayonnement modifié.

Ainsi, pour chaque position angulaire (dans le plan de rotation) de l'ensemble formé du générateur et du capteur, ce dernier reçoit un rayonnement porteur d'informations caractéristiques des zones de l'objet illuminées par ce rayonnement et transforme le rayonnement reçu en un signal qui est représentatif d'une image radiographique plane de l'objet illuminé (image radiographique 3D de l'objet projetée dans un plan) dans la position angulaire considérée.

On appelle une projection le signal ainsi fourni par le capteur.

Dans l'exemple décrit, l'ensemble du dispositif formé par le générateur et le capteur tourne, par exemple, d'un degré de rotation à chaque mouvement (pas) de la structure rotative porteuse et une projection est obtenue pour chaque degré de rotation dans cet exemple.

On notera qu'un tour complet est nécessaire avec le capteur décalé selon l'invention pour acquérir une quantité suffisante de données aux fins de reconstruction volumique de l'objet.

Ces projections ou signaux sont transféré(e)s au fur et à mesure de leur obtention (ou bien en une seule fois) à une unité de traitement des données.

Cette unité de traitement de données peut être éloignée de l'appareil de radiologie 10, à une distance de l'ordre de plusieurs mètres ou à plus grande distance.

L'unité de traitement de données est, par exemple, un ordinateur tel qu'un ordinateur PC dans lequel sont stockés un ou plusieurs fichiers informatiques contenant un algorithme de reconstruction de l'objet ou d'une zone d'intérêt de celui-ci en trois dimensions.

L'algorithme de reconstruction qui va être appliqué aux données provenant du capteur tient compte du décalage du capteur dans les opérations de traitement qu'il prévoit.

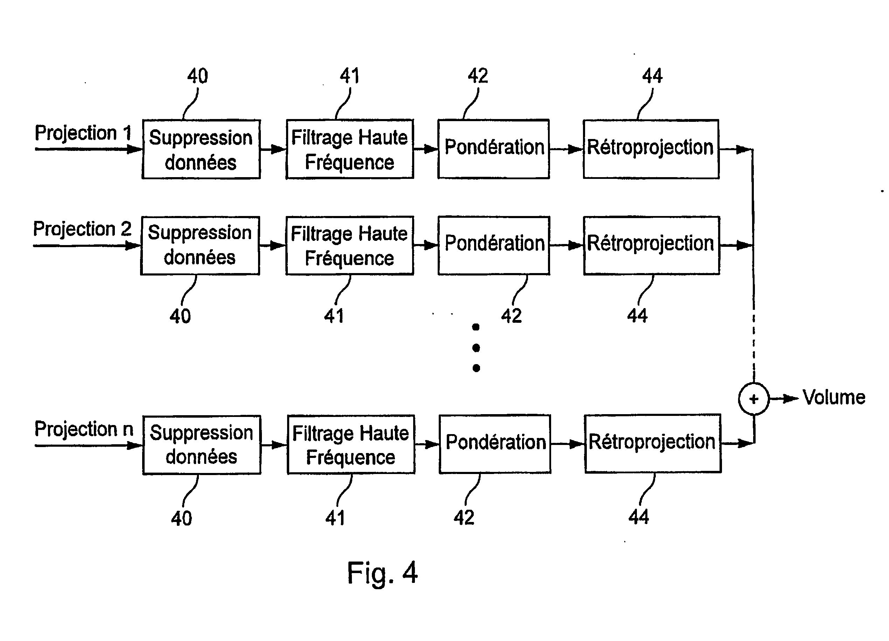

La figure 4 illustre de façon schématique des opérations de traitement prévues par un algorithme de reconstruction du type algorithme FDK ou algorithme de Feldkamp appliqué à chacune des projections délivrées par le capteur.

Cet algorithme connu en soi a été modifié afin de s'adapter au décalage précité.

Pour pouvoir reconstruire le volume de l'objet (par exemple, demi-arcade dentaire ou dent) le capteur décalé selon l'invention doit effectuer une rotation de 360°, là où, avec un capteur de largeur double et non décalé, une rotation de 180° suffit. De ce fait, à pas angulaire donné, on dispose de moins de vues avec le capteur décalé et le bruit de reconstruction est donc plus élevé.

Afin de limiter les effets de ce bruit, l'algorithme illustré à la figure 4 comprend avantageusement un premier bloc de traitement 40 qui prévoit d'enlever, de chaque projection 1 à n obtenues successivement au cours des rotations successives du capteur et du générateur, les données recueillies par la zone périphérique 20c du capteur et qui ne concernent pas l'objet puisqu'elles entourent seulement celui-ci. La position de cette zone est connue dès la mise en place de l'appareil dans la nouvelle configuration et, dès lors, la suppression des données correspondantes par calcul est aisée. On notera qu'en réduisant le volume de données à traiter par l'algorithme aux blocs suivants, on réduit le volume de calcul et donc le temps de calcul.

L'algorithme comprend ensuite un deuxième bloc de traitement 41 qui prévoit d'effectuer un filtrage haute fréquence particulier des données fournies par le capteur sur chacune des projections 1 à n après suppression des données de la zone 20c.

De façon générale, le filtrage effectué par le bloc 41 permet de différencier le bruit associé aux signaux recueillis par le capteur de l'information utile présente dans ces signaux en décomposant de façon indépendante les différentes bandes de fréquence qui sont dans les signaux. En effet, la géométrie décalée a introduit un bruit de reconstruction.

Chaque signal ou projection est donc filtré(e) par le bloc 41 où il subit un filtrage multiéchelle à décomposition de type pyramidale en bandes de fréquence. Plus particulièrement, il s'agit d'un filtrage par décomposition du type différence de gaussiennes. L'algorithme qui est mis en oeuvre dans ce bloc 41 est itératif et fournit, pour chaque bande de fréquence du signal considéré, un filtrage pyramidal haute fréquence de type rampe.

Plus particulièrement, l'opération effectuée dans ce bloc consiste en plusieurs étapes successives, au cours desquelles :
- l'image (projection) est sous-échantillonnée suivant une puissance de 2 dans le sens horizontal et suivant un facteur 1 dans le sens vertical,
- une différence de gaussiennes monodimensionnelles est appliquée à chaque échantillon précédemment obtenu,
- le résultat de l'étape précédente est combiné de façon pondérée à l'image haute fréquence de l'étage inférieur qui aura été préalablement sur-échantillonnée par deux. Les lois de pondération sont ajustées de façon à discriminer le bruit de l'information utile.

Les étapes sont réitérées autant de fois que la taille de l'image (projection) permet de contenir des puissances de 2.

On obtient ainsi en sortie du bloc 41 le résultat de tous les filtrages effectués sur le signal, de la fréquence la plus basse à la fréquence la plus haute.

Cette étape permet d'identifier facilement parmi les données les composantes en bruit et les fréquences d'intérêt sur chaque signal.

Cette discrimination entre le bruit et les fréquences utiles est en outre effectuée moyennant un temps de calcul rapide du fait du processus itératif.

L'algorithme de la figure 4 comporte avantageusement un troisième bloc de traitement 42 appliqué aux données issues du bloc 41 et destiné à homogénéiser la zone de recouvrement avec les zones de non recouvrement de l'objet radiographié.

De façon générale, chaque signal ou projection filtré dans le bloc 41 est multiplié, dans le bloc 42, par une fonction de pondération qui tient compte des redondances existant entre les données issues des différents signaux ou projections, compte tenu de la zone de recouvrement 39 (partie ou zone de l'objet illuminée en permanence par le faisceau de rayons X lors de sa rotation).

De façon générale, la pondération est ajustée en fonction de la localisation, par rapport à la zone de recouvrement 39 (figure 3e), des parties de l'objet illuminées par le faisceau de rayons X au cours des rotations successives du capteur et du générateur et qui ont donné lieu successivement aux différents signaux fournis par le capteur.

La fonction de pondération appliquée dans le bloc 42 est continue et régulière. Plus particulièrement, cette fonction varie entre 100 % et 0 % du bord de l'image correspondant aux pixels du capteur 20 les plus éloignés de l'axe longitudinal 34 au bord de l'image correspondant aux pixels du capteur les plus proches de cet axe.

En particulier, la fonction de pondération vaut, par exemple, ½ sur l'axe de rotation 30.

Ainsi pondérés, les projections filtrées sont traitées par le bloc suivant 44 qui effectue une rétroprojection de ces projections.

Cette étape de rétroprojection connue de l'algorithme conventionnel FDK effectue une rétroprojection de chaque projection filtrée et pondérée afin de reconstituer chaque voxel (unité élémentaire de volume qui est directement liée à la taille du pixel ramené dans le plan de l'objet ou de la zone d'intérêt) de l'objet radiographié ou d'une zone d'intérêt de celui-ci.

Plus particulièrement, au cours de cette étape, on attribue à tous les voxels situés sur le chemin des rayons X considérés une valeur qui dépend de la valeur du pixel atteint par ces rayons dans la projection considérée.

On répète ces quatre opérations des blocs respectifs 40, 41, 42, 44 pour chaque projection en ajoutant le résultat de l'opération de rétroprojection obtenu pour une projection donnée au volume déjà reconstruit à partir des rétroprojections précédentes.

On est ainsi en mesure de reconstruire l'objet ou une zone d'intérêt de celui-ci par tranches. On peut donc reconstituer une représentation volumique de l'objet ou d'une zone d'intérêt de celui-ci à partir d'un capteur de rayons X de dimensions réduites par rapport à la taille du capteur qui aurait été nécessaire en l'absence de décalage.

## Revendications

1. Appareil de radiologie dentaire du type à tomographie numérisée à faisceau conique, comprenant :
- un générateur de rayons X (18) apte à émettre un faisceau de rayons X vers un objet et qui est muni de moyens de collimation adaptés à collimater le faisceau émis,
- un capteur de rayons X ayant une surface active (20a) disposée en vis-à-vis du générateur, le générateur et le capteur étant aptes à se déplacer simultanément en rotation autour d'un axe de rotation (30),
**caractérisé en ce que** le capteur (20) est orienté de telle façon qu'un axe longitudinal (34) allant du générateur au capteur et passant par l'axe de rotation (30) est perpendiculaire à la surface active du capteur, le centre du capteur étant décalé transversalement par rapport à la projection de l'axe (34) sur la surface active du capteur, l'agencement des moyens de collimation et du capteur ainsi décalé étant tel que le faisceau collimaté illumine la surface active dudit capteur en laissant une zone périphérique de ladite surface faiblement illuminée par le faisceau collimaté par rapport au reste de la surface active, de manière à assurer une fonction de radioprotection vis-à-vis du rayonnement X.

2. Appareil selon la revendication 1, **caractérisé en ce que** le capteur et le générateur sont aptes à se déplacer en rotation dans un plan dit de rotation qui est traversé par l'axe de rotation (30).

3. Appareil selon la revendication 2, **caractérisé en ce que** le centre (20b) du capteur est positionné transversalement par rapport à la projection de l'axe longitudinal (34) sur la surface active du capteur à une distance qui, mesurée dans le plan de rotation, est au maximum égale à la différence entre la demi-largeur du capteur et une largeur suffisante pour laisser la zone périphérique de la surface active du capteur faiblement illuminée par rapport au reste de la surface active, la largeur du capteur étant la dimension mesurée dans le plan de rotation perpendiculairement à l'axe longitudinal (34).

4. Appareil selon la revendication 3, **caractérisé en ce que** le centre du capteur est positionné à une distance comprise entre le quart de la largeur du capteur et la distance maximale.

5. Appareil selon l'une des revendications 1 à 4, **caractérisé en ce que** l'agencement des moyens de collimation et du capteur est tel que le faisceau collimaté illuminant la surface active du capteur est délimité par un bord qui est placé au plus prés de la projection de l'axe (34) sur la surface active, à une distance minimale qui permet d'obtenir une zone de recouvrement minimale lors de la rotation du générateur et du capteur.

6. Appareil selon l'une des revendications 1 à 5, **caractérisé en ce que** la pente d'anode (19) du générateur (18) est modifiée en fonction de la position décalée du capteur afin de rendre plus uniforme le profil du rayonnement illuminant la surface active du capteur ainsi décalé.

7. Appareil selon la revendication 6, **caractérisé en ce que** l'angle (α) formé par la pente d'anode (19) du générateur et l'axe longitudinal (34) est ouvert dans la direction vers laquelle le capteur est décalé.

8. Procédé de reconstitution d'une représentation volumique d'un objet irradié par des rayons X à partir d'images radiographiques dentaires planes, ledit procédé faisant intervenir un générateur de rayons X (18) et un capteur de rayons X (20) qui sont aptes à se déplacer simultanément en rotation autour d'un axe de rotation (30), le procédé comprenant les étapes suivantes :
- émission d'un faisceau de rayons X vers un objet à partir du générateur de rayons X et collimation de ce faisceau par des moyens de collimation,
- réception, par le capteur, du faisceau de rayons X collimaté ayant irradié l'objet, **caractérisé en ce que** le faisceau de rayons X est collimaté sur la surface active du capteur dont le centre est décalé transversalement par rapport à la projection, sur la surface active du capteur, d'un axe longitudinal (34) allant du générateur au capteur et passant par l'axe de rotation, une zone périphérique de la surface active du capteur ainsi décalé étant faiblement illuminée par le faisceau collimaté par rapport au reste de la surface active, de manière à assurer une fonction de radioprotection vis-à-vis du rayonnement X.

9. Procédé selon la revendication 8, **caractérisé en ce que** le capteur et le générateur sont aptes à se déplacer en rotation dans un plan dit de rotation qui est traversé par l'axe de rotation (30).

10. Procédé selon la revendication 9, **caractérisé en ce que** le centre (20b) du capteur est positionné transversalement par rapport à la projection de l'axe longitudinal (34) sur la surface active du capteur à une distance qui, mesurée dans le plan de rotation, est au maximum égale à la différence entre la demi-largeur du capteur et une largeur suffisante pour laisser la zone périphérique de la surface active du capteur faiblement illuminée par rapport au reste de la surface active, la largeur du capteur étant la dimension mesurée dans le plan de rotation perpendiculairement à l'axe longitudinal (34).

11. Procédé selon la revendication 10, **caractérisé en ce que** le centre du capteur est positionné à une distance comprise entre le quart de la largeur du capteur et la distance maximale.

12. Procédé selon l'une des revendications 8 à 11, **caractérisé en ce que** l'agencement des moyens de collimation et du capteur est tel que le faisceau collimaté illuminant la surface active du capteur est délimité par un bord qui est placé au plus près de la projection de l'axe (34) sur la surface active, à une distance minimale qui permet d'obtenir une zone de recouvrement minimale lors de la rotation du générateur et du capteur.

13. Procédé selon l'une des revendications 8 à 12, **caractérisé en ce qu'**il comporte une étape de déplacement simultané du capteur (20) et du générateur (18) en rotation autour de l'axe de rotation (30), dans plusieurs positions angulaires successives, et pour chacune de ces positions angulaires successives, le procédé comporte une étape de fourniture, par le capteur, d'un signal représentatif d'une image radiographique plane de l'objet irradié suivant cette position angulaire, l'ensemble des signaux fournis par le capteur pour l'ensemble des positions angulaires successives contenant la totalité des données nécessaires à la reconstruction d'une représentation volumique de l'objet.

14. Procédé selon la revendication 13, **caractérisé en ce qu'**il comporte une étape de traitement des signaux fournis par le capteur pour l'ensemble des positions angulaires successives afin de reconstruire la représentation volumique de l'objet.

15. Procédé selon la revendication 14, **caractérisé en ce que** le traitement comprend une étape de pondération des données qui sont issues des différents signaux et proviennent des parties de l'objet successivement illuminées par le faisceau de rayons X au cours des rotations successives, la pondération étant ajustée en fonction de la présence ou non des parties illuminées de l'objet dans une zone de ce dernier appelée zone de recouvrement et qui est toujours illuminée par le faisceau au cours des rotations successives.

## Claims

1. Dental X-ray apparatus of the cone beam computed tomography type, comprising:
- an X-ray generator (18) adapted to emit a beam of X-rays toward an object and provided with collimation means adapted to collimate the emitted beam,
- an X-ray sensor having an active surface (20a) disposed facing the generator, the generator and the sensor being adapted to be moved simultaneously in rotation about a rotation axis (30),
**characterized in that** the sensor (20) is oriented in such a manner that a longitudinal axis (34) extending from the generator to the sensor and passing through the rotation axis (30) is perpendicular to the active surface of the sensor, the center of the sensor being offset transversely relative to the projection of the axis (34) onto the active surface of the sensor, and the arrangement of the collimation means and the sensor offset in this way being such that the collimated beam illuminates the active surface of said sensor leaving a peripheral area of said surface weakly illuminated by the collimated beam compared to the rest of the active surface so as to provide an X-ray radioprotection function.

2. Apparatus according to claim 1, **characterized in that** the sensor and the generator are adapted to be moved in rotation in a rotation plane through which the rotation axis (30) passes.

3. Apparatus according to claim 2, **characterized in that** the center (20b) of the sensor is positioned transversely relative to the projection of the longitudinal axis (34) on the active surface of the sensor at a distance which, measured in the rotation plane, is at most equal to the difference between the half-width of the sensor and a width sufficient to leave the peripheral area of the active surface of the sensor weakly illuminated compared to the rest of the active surface, the width of the sensor being the dimension measured perpendicularly to the longitudinal axis (34) in the rotation plane.

4. Apparatus according to claim 3, **characterized in that** the center of the sensor is positioned at a distance between one quarter of the width of the sensor and the maximum distance inclusive.

5. Apparatus according to any of claims 1 to 4, **characterized in that** the arrangement of the collimation means and the sensor is such that the collimated beam illuminating the active surface of the sensor is delimited by an edge that is placed as close as possible to the projection of the axis (34) onto the active surface at a minimum distance that makes it possible to obtain a minimum overlap area during rotation of the generator and the sensor.

6. Apparatus according to any of claims 1 to 5, **characterized in that** the anode slope (19) of the generator (18) is modified as a function of the offset position of the sensor in order to render more uniform the profile of the radiation illuminating the active surface of the sensor offset in this way.

7. Apparatus according to claim 6, **characterized in that** the angle (α) between the anode slope (19) of the generator and the longitudinal axis (34) is open in the direction toward which the sensor is offset.

8. Method of reconstitution of a three-dimensional representation of an object irradiated by X-rays from plane dental radiographic images, said method employing an X-ray generator (18) and an X-ray sensor (20) that are adapted to be moved simultaneously in rotation about a rotation axis (30), the method comprising the following steps:
- emission of a beam of X-rays toward an object from the X-ray generator and collimation of this beam by collimation means,
- reception by the sensor of the collimated beam of X-rays having irradiated the object, **characterized in that** the beam of X-rays is collimated onto the active surface of the sensor the center of which is offset transversely relative to the projection on the active surface of the sensor of a longitudinal axis (34) extending from the generator to the sensor and passing through the rotation axis, a peripheral area of the active surface of the sensor offset in this way being weakly illuminated by the collimated beam compared to the rest of the active surface so as to provide an X-ray radioprotection function.

9. Method according to claim 8, **characterized in that** the sensor and the generator are adapted to be moved in rotation in a rotation plane through which the rotation axis (30) passes.

10. Method according to claim 9, **characterized in that** the center (20b) of the sensor is positioned transversely relative to the projection of the longitudinal axis (34) onto the active surface of the sensor at a distance which, measured in the rotation plane, is at most equal to the difference between the half-width of the sensor and a width sufficient to leave the peripheral area of the active surface of the sensor weakly illuminated compared to the rest of the active surface, the width of the sensor being the dimension measured in the rotation plane perpendicularly to the longitudinal axis (34).

11. Method according to claim 10, **characterized in that** the center of the sensor is positioned at a distance between one quarter of the width of the sensor and the maximum distance inclusive.

12. Method according to any of claims 8 to 11, **characterized in that** the arrangement of the collimation means and the sensor is such that the collimated beam illuminating the active surface of the sensor is delimited by an edge that is placed as close as possible to the projection of the axis (34) onto the active surface at a minimum distance that makes it possible to obtain a minimum overlap area during rotation of the generator and the sensor.

13. Method according to any of claims 8 to 12, **characterized in that** it includes a step of simultaneous movement of the sensor (20) and the generator (18) in rotation about the rotation axis (30) in a plurality of successive angular positions and the method includes for each of these successive angular positions a step of the sensor providing a signal representative of a plane radiographic image of the irradiated object **in that** angular position, all the signals provided by the sensor for all the successive angular positions containing all the data necessary for the reconstruction of a representation in three dimensions of the object.

14. Method according to claim 13, **characterized in that** it includes a step of processing the signals supplied by the sensor for all successive angular positions in order to reconstruct the representation of the object in three dimensions.

15. Method according to claim 14, **characterized in that** the processing includes a step of weighting the data from the various signals and coming from parts of the object successively illuminated by the beam of X-rays during the successive rotations, the weighting being adjusted as a function of the presence or non-presence of the illuminated parts of the object in an area of the latter called the overlap area that is always illuminated by the beam during successive rotations.

## Patentansprüche

1. Dentalröntgengerät des Typs mit digitalisierter Tomographie mit konischem Strahlenbündel, das umfasst:
- einen Röntgenstrahlgenerator (18), der ein Röntgenstrahlbündel zu einem Objekt aussenden kann und der mit Kollimationsmitteln ausgestattet ist, die dazu ausgelegt sind, das ausgesendete Strahlenbündel zu kollimieren,
- einen Röntgenstrahlsensor mit einer aktiven Oberfläche (20a), die gegenüber dem Generator angeordnet ist, wobei der Generator und der Sensor sich gleichzeitig rotatorisch um eine Drehachse (30) verlagern können,
**dadurch gekennzeichnet, dass** der Sensor (20) derart orientiert ist, dass eine Längsachse (34), die vom Generator zum Sensor verläuft und durch die Drehachse (30) verläuft, zur aktiven Oberfläche des Sensors senkrecht ist, wobei das Zentrum des Sensors in Bezug auf die Projektion der Achse (34) auf die aktive Oberfläche des Sensors quer versetzt ist, wobei die Anordnung der Kollimationsmittel und des so versetzten Sensors derart ist, dass das kollimierte Strahlenbündel die aktive Oberfläche des Sensors beleuchtet, wobei eine Umfangszone der Oberfläche durch das kollimierte Strahlenbündel in Bezug auf den Rest der aktiven Oberfläche schwach beleuchtet belassen wird, um eine Strahlenschutzfunktion gegenüber der Röntgenstrahlung sicherzustellen.

2. Gerät nach Anspruch 1, **dadurch gekennzeichnet, dass** sich der Sensor und der Generator in einer so genannten Drehebene rotatorisch verlagern können, die von der Drehachse (30) durchquert wird.

3. Gerät nach Anspruch 2, **dadurch gekennzeichnet, dass** das Zentrum (20b) des Sensors in Bezug auf die Projektion der Längsachse (34) auf die aktive Oberfläche des Sensors in einem Abstand quer angeordnet ist, der in der Drehebene gemessen maximal gleich der Differenz zwischen der halben Breite des Sensors und einer Breite ist, die ausreicht, um die Umfangszone der aktiven Oberfläche des Sensors in Bezug auf den Rest der aktiven Oberfläche schwach beleuchtet zu belassen, wobei die Breite des Sensors die in der Drehebene senkrecht zur Längsachse (34) gemessene Abmessung ist.

4. Gerät nach Anspruch 3, **dadurch gekennzeichnet, dass** das Zentrum des Sensors in einem Abstand zwischen dem Viertel der Breite des Sensors und dem maximalen Abstand angeordnet ist.

5. Gerät nach einem der Ansprüche 1 bis 4, **dadurch gekennzeichnet, dass** die Anordnung der Kollimationsmittel und des Sensors derart ist, dass das kollimierte Strahlenbündel, das die aktive Oberfläche des Sensors beleuchtet, durch einen Rand begrenzt ist, der möglichst nahe an der Projektion der Achse (34) auf die aktive Oberfläche, in einem minimalen Abstand angeordnet ist, der es ermöglicht, eine minimale Abdeckungszone bei der Drehung des Generators und des Sensors zu erhalten.

6. Gerät nach einem der Ansprüche 1 bis 5, **dadurch gekennzeichnet, dass** die Neigung der Anode (19) des Generators (18) in Abhängigkeit von der versetzten Position des Sensors modifiziert wird, um das Profil der Strahlung, die die aktive Oberfläche des so versetzten Sensors beleuchtet, gleichmäßiger zu machen.

7. Gerät nach Anspruch 6, **dadurch gekennzeichnet, dass** der Winkel (α), der von der Neigung der Anode (19) des Generators und der Längsachse (34) gebildet wird, in der Richtung offen ist, in die der Sensor versetzt ist.

8. Verfahren zur Rekonstruktion einer Volumendarstellung eines mit Röntgenstrahlen bestrahlten Objekts aus ebenen Dentalröntgenbildern, wobei das Verfahren einen Röntgenstrahlgenerator (18) und einen Röntgenstrahlsensor (20) einsetzt, die sich gleichzeitig rotatorisch um eine Drehachse (30) verlagern können, wobei das Verfahren die folgenden Schritte umfasst:
- Aussenden eines Röntgenstrahlbündels zu einem Objekt vom Röntgenstrahlgenerator und Kollimation dieses Strahlenbündels durch Kollimationsmittel,
- Empfangen des kollimierten Röntgenstrahlbündels, das das Objekt bestrahlt hat, durch den Sensor, **dadurch gekennzeichnet, dass** das Röntgenstrahlbündel auf die aktive Oberfläche des Sensors kollimiert wird, dessen Zentrum in Bezug auf die Projektion einer Längsachse (34), die vom Generator zum Sensor verläuft und durch die Drehachse verläuft, auf die aktive Oberfläche des Sensors quer versetzt ist, wobei eine Umfangszone der aktiven Oberfläche des so versetzten Sensors durch das kollimierte Strahlenbündel in Bezug auf den Rest der aktiven Oberfläche schwach beleuchtet wird, um eine Strahlenschutzfunktion gegenüber der Röntgenstrahlung sicherzustellen.

9. Verfahren nach Anspruch 8, **dadurch gekennzeichnet, dass** sich der Sensor und der Generator in einer so genannten Drehebene rotatorisch verlagern können, die von der Drehachse (30) durchquert wird.

10. Verfahren nach Anspruch 9, **dadurch gekennzeichnet, dass** das Zentrum (20b) des Sensors in Bezug auf die Projektion der Längsachse (34) auf die aktive Oberfläche des Sensors in einem Abstand quer angeordnet ist, der in der Drehebene gemessen maximal gleich der Differenz zwischen der halben Breite des Sensors und einer Breite ist, die ausreicht, um die Umfangszone der aktiven Oberfläche des Sensors in Bezug auf den Rest der aktiven Oberfläche schwach beleuchtet zu belassen, wobei die Breite des Sensors die in der Drehebene senkrecht zur Längsachse (34) gemessene Abmessung ist.

11. Verfahren nach Anspruch 10, **dadurch gekennzeichnet, dass** das Zentrum des Sensors in einem Abstand zwischen dem Viertel der Breite des Sensors und dem maximalen Abstand angeordnet ist.

12. Verfahren nach einem der Ansprüche 8 bis 11, **dadurch gekennzeichnet, dass** die Anordnung der Kollimationsmittel und des Sensors derart ist, dass das kollimierte Strahlenbündel, das die aktive Oberfläche des Sensors beleuchtet, durch einen Rand begrenzt ist, der möglichst nahe an der Projektion der Achse (34) auf die aktive Oberfläche in einem minimalen Abstand angeordnet ist, der es ermöglicht, eine minimale Abdeckungszone bei der Drehung des Generators und des Sensors zu erhalten.

13. Verfahren nach einem der Ansprüche 8 bis 12, **dadurch gekennzeichnet, dass** es einen Schritt zur gleichzeitigen rotatorischen Verlagerung des Sensors (20) und des Generators (18) um die Drehachse (30) in mehreren aufeinander folgenden Winkelpositionen umfasst und für jede von diesen aufeinander folgenden Winkelpositionen das Verfahren einen Schritt zum Liefern eines Signals durch den Sensor umfasst, das ein ebenes Röntgenbild des bestrahlten Objekts gemäß dieser Winkelposition darstellt, wobei die Gesamtheit der vom Sensor gelieferten Signale für die Gesamtheit der aufeinander folgenden Winkelpositionen die Gesamtheit der für die Rekonstruktion einer Volumendarstellung des Objekts erforderlichen Daten enthält.

14. Verfahren nach Anspruch 13, **dadurch gekennzeichnet, dass** es einen Schritt zur Verarbeitung der vom Sensor gelieferten Signale für die Gesamtheit der aufeinander folgenden Winkelpositionen umfasst, um die Volumendarstellung des Objekts zu rekonstruieren.

15. Verfahren nach Anspruch 14, **dadurch gekennzeichnet, dass** die Verarbeitung einen Schritt zur Gewichtung der Daten, die von den verschiedenen Signalen stammen und von den Teilen des Objekts stammen, die nacheinander durch das Röntgenstrahlenbündel im Verlauf der aufeinander folgenden Drehungen beleuchtet werden, umfasst, wobei die Gewichtung in Abhängigkeit von der Anwesenheit oder Nicht-Anwesenheit der beleuchteten Teile des Objekts in einer Zone dieses letzteren, die Abdeckungszone genannt wird und die im Verlauf der aufeinander folgenden Drehungen immer durch das Strahlenbündel beleuchtet wird, eingestellt wird.
